(19) Europäisches Patentamt
European Patent Office
Office européen des brevets

(11) **EP 2 022 564 A1**

(12) **EUROPEAN PATENT APPLICATION**

(43) Date of publication:
**11.02.2009 Bulletin 2009/07**

(51) Int Cl.:
***B01J 29/44*** (2006.01)   ***B01J 29/46*** (2006.01)
***C07C 4/18*** (2006.01)

(21) Application number: **07113573.5**

(22) Date of filing: **31.07.2007**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(71) Applicant: **SHELL INTERNATIONALE RESEARCH MAATSCHAPPIJ B.V.
2596 HR Den Haag (NL)**

(72) Inventor: **The designation of the inventor has not yet been filed**

(74) Representative: **Zeestraten, Albertus W. J.
Shell International B.V.
Intellectual Property Services
P.O. Box 384
2501 CJ The Hague (NL)**

(54) **Catalyst composition, its preparation and use**

(57) A catalyst composition which comprises: a) a carrier which comprises a pentasil zeolite of at least 20wt% on the basis of the total carrier and being in its H+ form; b) platinum in an amount in the range of from 0.001 to 0.1 wt%, on the basis of the total catalyst; and, c) at least one additional metal selected from: nickel, copper and silver in an amount in the range from 0.0001 to 0.1 wt% on the basis of the total catalyst. The catalyst is typically used in an ethylbenzene dealkylation process of a hydrocarbonaceous feed and can reduce transalkylation activity and the consequential unwanted loss of xylene within the feed. Certain especially preferred embodiments containing silver can surprisingly increase benzene purity.

EP 2 022 564 A1

**Description**

Field of Invention

The present invention relates to a catalyst composition, its preparation, and its use in ethylbenzene dealkylation.

Background to the Invention

Ethylbenzene is one of the aromatic hydrocarbons that is obtained from naphtha pyrolysis or in reformate. Reformate is an aromatic product given by the catalysed conversion of straight-run hydrocarbons boiling in the 70 to 190°C range, such as straight-run naphtha. Such hydrocarbons are themselves obtained by the fractionation or distillation of crude petroleum oil, but generally having a low aromatics content. On conversion to reformate, the aromatics content is considerably increased and the resulting hydrocarbon mixture becomes highly desirable as a source of valuable chemicals intermediates and as a component for gasoline. The principle components are a group of aromatics often referred to as BTX: benzene, toluene, and the xylenes, including ethylbenzene. Other components may be present such as their hydrogenated homologues (e.g. cyclohexane).

Of the BTX group the most valuable components are benzene and the xylenes, therefore BTX is often subjected to processing to increase the proportion of those two aromatics: hydrodealkylation of toluene to benzene and toluene disproportionation to benzene and xylenes. Within the xylenes, para-xylene is the most useful commodity and xylene isomerisation or transalkylation processes have been developed to increase the proportion of para-xylene.

A further process that the gasoline producer can utilize is the hydrodealkylation of ethylbenzene to benzene.

Generally, the gasoline producer will isolate BTX from the reformate stream, and then subject the BTX stream to xylene isomerisation with the aim of maximising the para-xylene component. Xylene isomerisation is a catalytic process; some catalysts used in this process have the ability not just to isomerise xylenes but also simultaneous to dealkylate the ethylbenzene component. Normally, the para-xylene is then separated out to leave benzene, toluene (unless toluene conversion processes have already been applied) and the remaining mixed xylenes, including ethylbenzene.

The BTX stream can either be converted by transalkylation to increase the yield of xylenes by contacting with heavier hydrocarbon streams or can be converted by dealkylation to eliminate selectively ethylbenzene and to increase the yield of benzene, while allowing the xylenes to reach equilibrium concentrations. The latter process is the subject of the present invention.

In ethylbenzene dealkylation of the BTX stream, it is a primary concern to ensure not just a high degree of conversion to benzene but also to avoid xylene loss. Xylenes may typically be lost due to transalkylation, e.g. between benzene and xylene to give toluene, or by addition of hydrogen to form, for example, alkenes or alkanes.

It is therefore an aim of the present invention to provide a catalyst that will convert ethylbenzene to benzene with a reduced transalkylation activity. Simultaneous xylene isomerisation to equilibrium concentrations is also desirable.

The catalyst used for the production of reformate are often platinum-on-alumina catalysts. A wide range of proposals utilising zeolitic catalysts have been made for the conversion of BTX streams to increase the proportion of closely configured molecules. These include EP-A-0 018 498, EP-A-0 425 712, and WO 00/38834.

European Patent Specification No. 018 498 A1 is concerned with catalysts suitable for xylene isomerisation and the simultaneous dealkylation of ethylbenzene and reviews a number of earlier proposals for the use of platinum ZSM-series zeolitic catalysts. Generally such catalysts are shown to have a superior activity in isomerising xylenes and to dealkylate ethylbenzene, but are required to be used at high temperatures as there is a tendency for platinum to hydrogenate the benzene ring at low temperatures that are preferred for xylene isomerisation. The proposal of EP-A-0 018 498 is to use a second metal, which is preferably tin, barium, titanium, indium and cadmium, in combination with the platinum and a high-silica zeolite bound with a refractory inorganic oxide, which in all of the examples is alumina.

EP-A-0 425 712 describes an improved catalyst for the simultaneous xylene isomerisation and ethylbenzene dealkylation, which is formed by combining a Group VIII metal, preferably platinum, with a lead component, and a halogen component, on a carrier of a pentasil zeolite and an inorganic oxide binder, preferably alumina, such that a specific ratio of lead to Group VIII metal is achieved and such that bulk of the Group VIII and the lead components are combined with the binder material.

WO 00/38834 describes a mixed zeolitic catalyst for the disproportionation and transalkylation of aromatic hydrocarbons. The catalyst consists of a carrier of 10 to 80 wt% mordenite and/or zeolite beta, 0 to 70 wt% ZSM-5, and 5 to 90 wt% inorganic binder, plus a metal component of platinum with either tin or lead. While the binder is said to be most preferably alumina or silica, only alumina-bound catalysts are exemplified.

There are fewer proposals for catalysts directed solely for the hydrodealkylation of aromatics.

Toppi at al in Journal of Catalysis 210, 431-444 (2002) studies the use of silica-supported platinum-tin catalysts in comparison with acidic catalysts of just alumina and chlorinated alumina, on the hydrodealkylation of n-propylbenzene, and finds that the formation rate of benzene was the highest for the acidic catalysts.

US-A-3, 992, 468 proposes two catalysts for the hydrodealkylation of alkylaromatic hydrocarbons: catalyst A essentially containing a) a carrier, b) at least one metal selected from the group consisting of the metals from group VIII and c) at least one metal selected from the group consisting of zinc, cadmium, gallium, indium, thallium, copper, silver, gold, yttrium, titanium, niobium, tantalum, and manganese; and catalyst B essentially containing a) a carrier, b) at least one metal selected from a first group consisting of chromium, molybdenum, tungsten, rhenium, and manganese, and c) at least one additional metal different to that of the first group plus copper, silver, gold, zinc, cadmium, gallium, indium, thallium, germanium, tin and lead, each metal being in an amount of from 0.05 to 20 wt%. The carrier is selected from among known carriers, for example, alumina, magnesia, magnesia-silica, zirconia, zirconia-silica, and molecular sieves or zeolites, and is preferably alumina.

EP- A- 1 495 805 discloses the use of an MFI type zeolite based catalyst containing a) alkaline earth metal, ideally calcium, strontium or barium, in the range of from 0.05 to 5.0 wt%, b) silver in the range of from 0.1 to 5 wt%, c) at least one metal selected from the group consisting rhenium, platinum, or nickel in the range of 0.005 to 1.5 wt%, where weights are in relation to the catalyst as a whole, for the catalytic conversion of ethylbenzene containing xylenes.

Summary of Invention

The present invention provides a catalyst composition which comprises:

a) a carrier which comprises a pentasil zeolite of at least 20wt% on the basis of the total carrier and being in its $H^+$ form;
b) platinum in an amount in the range of from 0.001 to 0.1 wt%, on the basis of the total catalyst; and,
c) at least one additional metal selected from: nickel, copper and silver in an amount in the range from 0.0001 to 0.1 wt% on the basis of the total catalyst.

The present invention also provides a process for the preparation of a catalyst as defined herein, which comprises combining at least 20 wt% of pentasil zeolite, shaping the resulting mixture, if desired, and compositing within the range of from 0.001 to 0.1 wt% platinum, and in the range of from 0.0001 to 0.1 wt% of at least one additional metal selected from: nickel, copper, or silver.

Also provided is an ethylbenzene dealkylation process which comprises contacting in the presence of hydrogen a feedstock which comprises ethylbenzene, with a catalyst composition as defined herein or as prepared by a process as defined herein.

Detailed description of the Invention

The catalyst composition of the present invention has been found to provide reduced xylene losses with lower transalkylation activity coupled with comparable benzene selectivity and benzene purity compared with an analogous mono-metallic catalyst.

Silica is preferably used as the binder in the catalyst composition of the present invention and may be a naturally occurring silica or may be in the form of a gelatinous precipitate, sol or gel. The form of silica is not limited and the silica may be in any of its various forms: crystalline silica, vitreous silica or amorphous silica. The term amorphous silica encompasses the wet process types, including precipitated silicas and silica gels, of pyrogenic or fumed silicas. Silica sols or colloidal silicas are non-settling dispersions of amorphous silicas in a liquid, usually water, typically stabilised by anions, cations, or non-ionic materials.

The silica binder is preferably a mixture of two silica types, most preferably a mixture of a powder form silica and a silica sol. Conveniently powder form silica has a surface area in the range of from 50 to 1000 $m^2$/g; and a mean particle size in the range of from 2 nm to 200μm, preferably in the range from 2 to 100μm, more preferably 2 - 60 μm especially 2 - 10 μm as measured by ASTM C 690-1992 or ISO 8130-1. A very suitable powder form silica material is Sipernat 50, a white silica powder having predominately spherical particles, available from Degussa (Sipernat is a trade name). A very suitable silica sol is that sold under the trade name of Bindzil by Eka Chemicals. Where the mixture comprises a powder form silica and a silica sol, then the two components may be present in a weight ratio of powder form to sol in the range of from 1:1 to 10:1, preferably from 2:1 to 5:1, more preferably from 2:1 to 3:1. The binder may also consist essentially of just the powder form silica.

Where a powder form of silica is used the binder in the catalyst composition of the present invention, preferably a small particulate form is utilised, which has a mean particle size in the range of from 2 to 10 μm as measured by ASTM C 690-1992. An additional improvement in carrier strength is found with such materials. A very suitable small particulate form is that available from Degussa under the trade name Sipernat 500LS.

The silica component used may be pure silica and not as a component in another inorganic oxide. For certain embodiments, the silica and indeed the carrier, is essentially free of any other inorganic oxide binder material, and especially is free of alumina. Optionally, at most only a maximum of 2 wt% alumina, based on the total carrier, is present.

For certain embodiments a surface modification treatment may be performed. For such embodiments, the presence of alumina can detrimentally affect the physical integrity of the carrier and so is less preferred.

Pentasil zeolites are well known to the skilled person. "Pentasil" is a term used to describe a class of shape-selective zeolites which are typically characterised by a silica alumina ratio (SAR) of at least 12 and are constructed of five-member rings (their framework being built up from 5-1 secondary building units). The pentasil zeolite utilised in the present invention has a SAR in the range of from 20 to 150. The SAR value is the bulk or overall silica/alumina ratio which may or may not be different to the framework SAR depending on any treatment to which the zeolite, either when free or in catalytic form, has been subjected.

Of the pentasil zeolites, the preferred zeolites are ZSM-5 (MFI type material), ZSM-12(MTW type material), ZSM-48 and ZSM-35 (FER type material), with ZSM-5 being the especially preferred. All four of these zeolites are known and documented in the literature, see for example http://www.iza-structure.org/databases/ or Baerlocher et al "Atlas of Zeolite framework types", 5th revised edition (2001), published on behalf of the Structure Commission of the International Zeolite Association, by Elsevier. Pentasils are reviewed in the Database at http://www.iza-structure.org/database/Cataloge/Pentasils.pdf.

Such zeolites can exist in various forms depending on the ion present at the cation sites in the zeolite structure. Generally the available forms contain an alkali metal ion, an alkaline earth metal ion, or a hydrogen or hydrogen precursor ion at the cation site. In the catalyst composition of the present invention, the zeolite is present in the form containing hydrogen or hydrogen precursor; this form is commonly known as the $H^+$ form. The zeolite may be used either in its template-free or its template-containing form. Some advantage in reduction of xylene loss can be expected where the template-containing form is used during the preparation.

The SAR of such zeolites is preferably at least 25, more preferably at least 30, and is preferably at most 100, more preferably at most 90, especially at most 50.

The zeolite starting material can exist in a number of particle size ranges. Suitably the zeolite has a primary particle diameter in the range of from 20 nm to 10 $\mu$m. Useful catalysts have been prepared using a large crystal size ZSM-5 zeolite having an average crystallite size in the range of from 1 to 10 $\mu$m, and also using a small particle size ZSM-5 having a primary particle diameter below 200 nm. Generally, in terms of particle size distributions, the ZSM-5 may have a particle size distribution in which the diameter of 50% of the particles is greater than 2 $\mu$m and that of 90% of the particles is less than 30 $\mu$m.

Suitable ZSM-5 materials can be prepared by procedure documented in the literature, for example in US-3, 702, 886, in references provided in the Atlas, or Database, of Zeolite Structures, and in other literature references such as by Yu et al in Microporous and Mesoporous Materials 95 (2006) 234 to 240, and Iwayama et al in US-A-4, 511, 547.

Suitable grades of ZSM-5 zeolite include CBV 3014E, CBV 3020E and CBV 8014, available commercially from Zeolyst International.

The zeolite is an important factor in the activity and selectivity properties shown by the catalyst composition of the invention. There is a balance between the activity and selectivity desired which may result in a different optimum zeolite content in the carrier depending on the zeolite used and the SAR value of the zeolite used. Generally a higher zeolite content may in some cases be advantageous to produce a higher activity from the catalyst composition, while a lower zeolite content may provide a higher selectivity.

It has been found that when using a ZSM-5 zeolite of SAR 30, reduction of the zeolite content gives rise to an increased benzene selectivity with lower xylene losses but for a penalty of a lower activity, as shown by a higher temperature required to provide the same level of conversion of ethylbenzene. Where a higher SAR zeolite is utilised it is necessary to increase the proportion of zeolite in the catalyst carrier to achieve optimum performance.

While this balance may cause a different optimum depending on the conditions utilised in the ethylbenzene dealkylation process, generally it is preferred to minimise the amount of zeolite used in the catalyst carrier, since a higher amount of zeolite may negatively affect the physical properties of the catalyst carrier such as lowering its strength. It is generally preferred that the carrier is composed of less than 70wt% zeolite, preferably less than 50 wt% and preferably more than 20, more preferably more than 30 wt%. The carrier composition preferably contains silica in the range of from 30 to 80 wt%, and more preferably from 50 to 70 wt%.

A very suitable catalyst carrier for the present invention contains a pentasil zeolite, especially ZSM-5, having a SAR in the range of from 20 to 50, especially 20 to 40, in an amount in the range of from 20 to 50 wt%, especially 25 to 45 wt%

For certain embodiments, there is no other component than silica and pentasil zeolite in the carrier. For alternative embodiments, other components may be included whilst still obtaining the benefits of the present invention. Such other components may be selected from other refractory inorganic oxide binder materials and other zeolites. Other binder materials may be titania, zirconia, alumina, silica-alumina and magnesia; preferably titania, zirconia and silica, especially silica. Alumina and alumina-silica are less preferred. For embodiments where surface modification is performed, preferably less than 10% of the binder is a binder other than silica.

Examples of other zeolites are 8, 10, or 12-membered ring zeolites, for example mordenite, and silica beta, and acidic mesoporous materials such as the MCM-series of zeolites, e.g. MCM-22 and MCM-41.

The carrier is conveniently a shaped carrier and may be treated to enhance the activity of the zeolite component. It has been found advantageous to perform a surface modification as described in US-B2-6, 949, 181.

Modification of the molecular sieve reduces the mole percentage of alumina which basically implies that the number of acid sites is reduced. This can be achieved in various ways. A first way is applying a coating of a low acidity inorganic refractory oxide onto the surface of the crystallites of the molecular sieve.

Another very useful way of modifying the molecular sieve is by subjecting it to a dealumination treatment. In general, dealumination of the crystallites of a molecular sieve refers to a treatment, whereby aluminium atoms are either withdrawn from the molecular sieve framework leaving a defect or are withdrawn and replaced by other atoms, such as silicon, titanium, boron, germanium, or zirconium.

In US Patent No. 5, 242, 676, a very suitable method for the dealumination of the surface of zeolite crystallites is disclosed. Another method for obtaining a zeolite having a dealuminated outer surface is disclosed in US Patent No. 4, 088, 605.

Of the (surface) dealumination methods described above, the method involving the treatment with a hexafluorosilicate, most suitably ammoniumhexa-fluorosilicate (AHS) as described in US-B2-6, 949, 181, may be expected to offer additional advantage.

In shaped form, for example as extrudates, the carrier generally has a surface area falling in the range of from 100 to 400 $m^2/g$, preferably 130 to 300 $m^2/g$, more preferably from 150 to 250 $m^2/g$; and a pore volume, by mercury intrusion, in the range of from 0.2 to 1.2 ml/g, preferably 0.4 to 1.0 ml/g, more preferably 0.5 to 0.9 ml/g.

The catalyst composition of the invention contains metal components in the form of platinum and an addition metal in the form of either copper, nickel, or silver, preferably silver. The platinum component is present in an amount in the range of from 0.001 to 0.1 wt%, based on total catalyst, and the additional metal component of copper, nickel or silver is present in the range of from 0.0001 to 0.1 wt%, based on the total catalyst. Preferably the additional metal component is less than 0,1wt%. Most suitably the platinum content is present in an amount in the range of from 0.01 to 0.1 wt%, and preferably from 0.01 to 0.05 wt%. The additional metal component is most suitably present in the range from 0.001 to 0.1 wt%. Even more suitably the additional metal component is silver present in an amount 0.001 to 0.05 wt%, and preferably 0.001 to 0.03 wt% Percentage weights are based on the total catalyst.

The catalyst composition of the invention has properties similar to that of the carrier in surface area and pore volume.

The catalyst composition of the present invention may be prepared using standard techniques for combining the zeolite, binder and optional other carrier components; shaping; compositing with the metals components; and any subsequent useful process steps such as drying, calcining, and reducing.

The shaping may by into any convenient form such as powders, extrudates, pills and granules. In the present invention preference is given to shaping by extrusion. To prepare extrudates, commonly the pentasil zeolite will be combined with the binder, preferably silica, and if necessary a peptising agent, and mixed to form a dough or thick paste. The peptising agent may be any material that will change the pH of the mixture sufficiently to induce de-agglomeration of the solid particles. Peptising agents are well known and encompass inorganic acids, such as sulphuric acid or nitric acid, and organic acids such as formic acid, acetic acid, citric acid, oxalic acid, and propionic acid and alkali materials for example alkali hydroxides, alkali earth hydroxides, ammonia or organic amines. Such peptising agents are removed upon calcination and are not retained in the extrudates. Ammonia may be provided in any suitable form, for example via an ammonia precursor. Examples of ammonia precursors are ammonium hydroxide and urea. It is also possible for the ammonia to be present as part of the silica component, particularly where silica sol is used, though additional ammonia may still be needed to impart the appropriate pH change. The amount of ammonia present during extrusion may affect the pore structure of the extrudates which may provide advantageous properties. Suitably the amount of ammonia present during extrusion may be in the range of from 0 to 5 wt% based on total dry mixture, more preferably 0 to 3 wt%, preferably 0 - 1.9wt% on dry basis. It is preferably that a calcination step be carried out on the resultant extrudate prior to emplacement of the metal components, this is preferably carried out at temperatures above 500°C and typically above 600°C.

The metals' emplacement onto the formed carrier may be by methods usual in the art. The metals can be deposited onto the carrier materials prior to shaping, but it is preferred to deposit them onto a shaped carrier.

Pore volume impregnation of the metals from a metal salt solution is a very suitable method of metals emplacement onto a shaped carrier. The metal salt solutions may have a pH in the range of from 1 to 12. The platinum salts that may conveniently be used are chloroplatinic acid and ammonium stabilised platinum salts. The additional silver, nickel or copper metal salt will typically be added in the form of water soluble organic or inorganic salt in solution. Examples of suitable salts are nitrates, sulphates, hydroxides and ammonium (amine) complexes.

The metals may be impregnated either sequentially or simultaneously. It is preferable that the metals be added simultaneously. Where simultaneous impregnation is utilised the metal salts used must be compatible and not hinder the deposition of the metals. It may be useful to utilise a complexing agent or chelating agent in a combined bimetallic salt solution to prevent unwanted metals precipitation. Examples of suitable complexing agents are EDTA (ethlyenediaminetetraacetic acid), and derivatives thereof; HEDTA (N-(2-hydroxyethyl)ethylenediamine-N,N',N'-triacetic acid), EGTA (ethylene glycol-bis(2-aminoethylether)-N,N,N',N'-tetraacetic acid), DTPA (diethylene tridiamine pentaacetic acid), and NTA (nitrilotriacetic acid). Where EDTA is used, it is conveniently used in a molar ratio to the additional metal of from 0.1 to 3, especially 1 to 2.

After shaping the carrier, subjecting it to a calcination step, and then metal impregnation, the carrier/catalyst composition is preferably dried, and calcined again. Drying temperatures are suitably 50 to 200°C; drying times are suitably from 0.5 to 5 hours. Calcination temperatures are very suitably in the range of from 200 to 800°C, preferably 300 to 600°C. For calcination of the carrier, a relatively short time is required, for example 0.5 to 3 hours. For calcination of the catalyst composition, it may be necessary to employ controlled temperature ramping at a low rate of heating to ensure the optimum dispersion of the metals: such calcination may require from 5 to 20 hours.

In a particularly preferred embodiment, the calcination temperature for the calcination step after metal impregnation (or deposition by other methods) is more than 500 °C, preferably more than 550 °C, typically less than 700 °C, preferably less than 650 °C, such as around 600 °C.

Prior to use, it is necessary to ensure that the metals on the catalyst composition are in metallic (and not oxidic) form. Accordingly, it is useful to subject the composition to reducing conditions, which are, for example, heating in a reducing atmosphere, such as in hydrogen optionally diluted by an inert gas, such as nitrogen or carbon dioxide, at temperature in the range of from 150 to 600°C for from 0.5 to 5 hours.

The catalyst composition of the invention finds especial use in the selective dealkylation of ethylbenzene.

The ethylbenzene feedstock most suitably originates directly from a reforming unit or naphtha pyrolysis unit or is the effluent of a xylene isomerisation unit. Such feedstock usually comprises $C_7$ to $C_9$ hydrocarbons, and in particular one or more of o-xylene, m-xylene, p-xylene, toluene, and benzene in addition to ethylbenzene. Generally the amount of ethylbenzene in the feedstock is in the range of from 0.1 to 50 wt% and the total xylene content is typically at least 20 wt%. Typically the xylenes will not be in a thermodynamic equilibrium, and the content of p-xylene will accordingly be lower than that of the other isomers.

The feedstock is contacted with the catalyst composition in the presence of hydrogen. This may be carried out in a fixed bed system, a moving bed system, or a fluidised bed system. Such systems may be operated continuously or in batch fashion. Preference is given to continuous operation in a fixed bed system. The catalyst may be used in one reactor or in several separate reactors in series or operated in a swing system to ensure continuous operation during catalyst change-out.

The process is suitably carried out at a temperature in the range of from 300 to 500°C, a pressure in the range of from 0.1 to 50 bar (10 to 5,000 kPa), using a liquid hourly space velocity of in the range of from 0.1 to 20 h$^{-1}$. The partial pressure of hydrogen in the range of from 0.05 to 30 bar (5 to 3,000 kPa) is generally used, and more preferably in the range of from 1 to 20 bar (100 to 2,000 kPa). The feed to hydrogen ratio mol.mol$^{-1}$ is in the range of from 0.5 to 100, generally from 1 to 10.

The present invention will now be illustrated by the following examples.

Examples

In the Examples and where mentioned elsewhere hereinabove, the following test methods are applicable: Porosity: ASTM D 4284 with drying of the sample at 300°C for 60 minutes prior to measurement, and using mercury intrusion. The pore volume and surface area is derived from mercury porosimetry.

In the Examples, unless otherwise specified, the zeolites were used in the H$^+$ form and free of template material.

Example 1 (Comparative)

Catalyst 1: mono-metallic platinum only sample

A carrier was prepared from a zeolite with a ZSM-5 structure with an average crystalline size below 1 $\mu$m and a silica to alumina bulk ratio (SAR) of 30. The zeolite powder was mixed with a low sodium grade silica (Sipernat 50 available from Degussa), and an ammonium stabilised commercially available silica sol (sold under the trade name Bindzil by Eka Chemicals).

The silica containing extrudates prepared from the carrier composition contained 30 wt% ZSM-5 zeolite. The extrudates were dried and calcined above 600°C to obtain a sufficient strength for industrial application.

The resulting carrier had a pore volume of 0.71 ml/g, measured by mercury porosimetry, and a surface area of 246 m$^2$/g derived from mercury porosimetry. The total aluminium content of the carrier was below 1wt%.

The carrier was pore volume impregnated with platinum salt solution, in excess of ammonia in the solution

(NH$_3$/Pt ratio higher than 20). Once the impregnation was completed, the catalyst was dried at 120°C for 2 hours, and subsequently calcined aiming at reaching a temperature of 480°C with a sufficiently low ramping rate to achieve adequate dispersion of the metallic phase. The total calcination procedure lasted 17 hours. The concentration of the platinum in the final extrudate catalyst has a Pt concentration of 0.02 wt% based on total catalyst.

Example 2

Catalyst 2: a bi-metallic catalyst containing platinum and nickel

A carrier composition identical to that used for Example 1 was used in an alternative catalyst preparation where an additional metal, in the form of nickel, is present at the impregnation stage. The obtained extrudates are subject to calcination at 480°C as in Example 1 and the resultant catalyst having a Pt concentration of 0.02 %wt, and a Ni concentration of 0.01%wt, based on total catalyst.

Example 3

Catalyst 3: a bi-metallic catalyst containing platinum and copper

A carrier composition identical to that used for Example 1 was used in an alternative catalyst preparation where an additional metal, in the form of copper, is present at the impregnation stage. The obtained extrudates are subject to calcination at 480°C as in Example 1 and the resultant catalyst having a Pt concentration of 0.02 %wt, and a Cu concentration of 0.01%wt, based on total catalyst.

Example 4

Catalyst 4: a bi-metallic catalyst containing platinum and silver

A carrier composition identical to that used for Example 1 was used in an alternative catalyst preparation where an additional metal, in the form of silver, is present at the impregnation stage. The obtained extrudates are subject to calcination at 480°C as in Example 1 and the resultant catalyst having a Pt concentration of 0.02 %wt, and an Ag concentration of 0.01%wt, based on total catalyst.

Example 5 (Comparative)

Catalyst 5: mono-metallic platinum only sample, higher calcination temperature

A catalyst was obtained following the procedure set out in Example 1 but was calcined at 600°C (as opposed to 480 °C) for 1 hour following the metal impregnation.

Example 6

Catalyst 6: bi-metallic catalyst containing platinum and copper, higher calcination temperature

A catalyst was obtained following the procedure set out in Example 3 but was calcined at 600°C (as opposed to 480 °C) for 1 hour following the metal impregnation.

Example 7

Catalyst 7: bi-metallic catalyst containing platinum and silver, higher calcination temperature

A catalyst was obtained following the procedure set out in Example 4 but was calcined at 600°C (as opposed to 480 °C) for 1 hour following the metal impregnation.

Example 8

Catalyst 8: bi-metallic catalyst containing platinum and copper

A carrier composition identical to that used for Example 1 was used in an alternative catalyst preparation where an additional metal, in the form of copper, is present at the impregnation stage. The obtained extrudates are subject to calcination at 480°C as in Example 1, and the resultant catalyst having a Pt concentration of 0.02 %wt, and a Cu concentration of 0.005%wt, based on total catalyst.

Example 9

Catalyst 9: bi-metallic catalyst containing platinum and copper

A carrier composition identical to that used for Example 1 was used in an alternative catalyst preparation where an additional metal, in the form of copper, is present at the impregnation stage. The obtained extrudates are subject to calcination at 480°C as in Example 1 and the resultant catalyst having a Pt concentration of 0.02 %wt, and a Cu concentration of 0.002%wt, based on total catalyst.

Activity testing of catalysts 1 to 9

The catalysts produced in Examples 1 to 9 were subjected to a catalytic activity micro-flow tests that mimic typical industrial application conditions for ethylbenzene dealkylation. This activity test uses an industrial feed of European origin.

Prior to the activity test the catalyst is loaded in a reactor with silicon carbide (SiC) dilution to ensure proper plug-flow conditions. The catalyst is then purged with nitrogen, followed by hydrogen. The activity test is performed once the catalyst is in its reduced state, which is achieved by exposing the dried and calcined catalyst to atmospheric hydrogen (> 99% purity) where the temperature is raised from room temperature to 450°C and maintained for 1 hour.

After reduction of the catalyst sample the reactor is pressurised to 13 bar (1,300 kPa), and without a cooling step and the feed is introduced. The feed is introduced at a space velocity of $10h^{-1}$. This step contributes to enhanced catalytic performance at stable operation.

After the pre-aging step, the catalytic activity is measured in an operating window of 360 to 410°C, with a space velocity of 3 to 6 $h^{-1}$, and a feed ratio $H_2$/HC in the range of 2 to 5 mol/mol, at a total pressure in the range of 6 to 12 bar (6 to 1,200 kPa). The composition of the feed used here is summarised in Table 1.

Table 1

| Composition of the feed used in the catalyst activity testing | | |
|---|---|---|
| Feed composition | | |
| EB | wt% | 10.66 |
| p-xylene | wt% | 0.08 |
| o-xylene | wt% | 18.79 |
| m-xylene | wt% | 65.17 |
| toluene | wt% | 0.92 |
| benzene | wt% | 0.00 |
| $C_7$ - $C_8$ - naphthenes | wt% | 4.38 |
| $C_9$+ aromatics | wt% | 0.00 |
| Total | wt% | 100.00 |
| | | |
| $C_8$ Aromatics | sum | 94.71 |
| EB in C8 aromatics feed | wt% | 11.25 |
| p-xylene in xylenes in feed | wt% | 0.10 |
| o-xylene in xylenes in feed | wt% | 22.36 |
| m-xylene in xylene in feed | wt% | 77.54 |

The catalytic data points are collected at a condition that exaggerates the potential negative operational effects. Therefore, the performance is measured not at the ideal industrial operating conditions, but at those that allow a better differentiation of the various performance parameters used to evaluate catalysts in this application.

The catalytic performance characteristics were evaluated as follows:

$$\text{EB conversion (wt\%)} = \frac{\text{EB}_\text{f.} - \text{EB}_\text{pr.}}{\text{EB}_\text{f.}} \times 100$$

$$\text{Benzene selectivity (mol\%)} = \frac{\text{B}_\text{pr.} - \text{B}_\text{f.}}{\text{EB}_\text{f.} - \text{EB}_\text{pr.}} \times \frac{106}{78} \times 100$$

$$\text{Benzene purity (wt\%)} = \frac{\text{B}_\text{pr.}}{\text{B}_\text{pr.} + \text{cHx}_\text{pr.} + \text{McP}_\text{pr.}} \times 100$$

$$\text{Xylene losses (wt\%)} = \frac{\text{Xyl}_\text{f.} - \text{Xyl}_\text{pr.}}{\text{Xyl}_\text{f.}} \times 100$$

$$\text{Toluene (wt\%)} = \left( \frac{\text{Tol}_\text{pr.}}{\sum \text{product}} - \frac{\text{Tol}_\text{f.}}{\sum \text{feed}} \right) \times 100$$

$$\text{C}_9^+ \text{aromatics (wt\%)} = \frac{\text{C}_9^+ \text{arom.}_\text{pr.}}{\sum \text{product}} \times 100$$

$$\text{Gas make (wt\%)} = \frac{\sum_{i=2}^{5} \text{C}_{i\text{pr.}}}{\sum \text{product}} \times 100$$

$$\text{pX ate (\%)} = \frac{\text{pX in Xyl}_\text{pr.} - \text{pX in Xyl}_\text{f.}}{\text{pX in Xyl}_\text{eq.} - \text{pX in Xyl}_\text{f.}} \times 100$$

Where EB stands for ethylbenzene, B for benzene, Tol for toluene, cHx for cyclohexane, McP for methyl cyclopentane, Xyl for xylenes in general (all isomers), pX for para-xylene, Cipr for all light hydrocarbons from $C_2$ to $C_5$ in the product, f for feed, and pr for product. The obtained data points can satisfactorily be fitted with a second order polynomial. The so obtained data polynomial is used for the determination of the temperature required for a given EB conversion.

Emphasising the relative differences between the performance indicators allows a much better evaluation of all performance parameters. For this, the difference in e.g. toluene (tol) made between catalyst 1 and catalyst 2 can be calculated as:

```
ΔY(tol)relative = (Y(tol)catA - Y(tol)catB )/ Y(tol)catB x 100
```

Where Y denotes yield. Similarly the relative differences of the xylene losses and $C_9^+$ yields can be calculated.

Xylene loss by transalkylation is an unwanted side reaction in an ethylbenzene dealkylation process. Toluene yield is a good indicator of transalkylation activity since it is not produced here by any other reaction. Therefore reduction in toluene yield is preferred. Other indicators of transalkylation activity are $C_9^+$ yields and xylene loss. Preferably $C_9^+$ yields and xylene loss are minimised.

The dealkylation reaction itself produces benzene and ethylene and so the benzene selectivity and purity is preferably maximised.

To determine benzene selectivity, and purity, another calculation approach is more suitable. Since these values are often close to 100, and it is desirable to increase the value as close as possible to 100, a ratio as defined for the transalkylation relative terms is unsuitable. Instead, the relative performance has to be defined as how far the value is from this desired 100% selectivity or purity as such:

$$\Delta S(B)_{relative} = (S(B)_{catA} - S(B)_{catB}) / (100 - S(B)_{catB}) \times 100$$

$$\Delta P(B)_{relative} = (P(B)_{catA} - P(B)_{catB}) / (100 - P(B)_{catB}) \times 100$$

Where S stands for selectivity, P for purity, both of benzene's

For all activity tests shown in the Examples 10 - 12 below, the activity of the catalyst is expressed as the $\Delta$ of the temperature required for 70 wt% ethylbenzene (EB) conversion versus the platinum only containing sample, catalyst 1.

Example 10

Activity Testing of Catalysts 1 to 4

A sample of each of the catalysts produced in Examples 1, 2, 3 and 4 were tested according to the activity testing method described above. The values obtained are shown on table 2.

Table 2

| Catalyst sample | $\Delta$ T required for 70% EB conversion (°C) |
|---|---|
| 1 (Pt) | 0.0 |
| 2 (Pt/Ni) | 2.7 |
| 3 (Pt/Cu) | 3.5 |
| 4 (Pt/Ag) | 1.0 |

The results clearly show that none of the catalyst samples tested lost significant activity upon modification to the bimetallic form, by the modification of the impregnation solution. The similarity of the temperatures required shows that the overall activities of these catalyst samples are closely comparable in terms of dealkylation. Thus activity is not significantly affected - changes of more than 10 °C would be significant.

Example 11

Activity Testing of Catalysts 5 to 7

Samples of the catalysts produced in Examples 5, 6 and 7 were tested according to the activity method as described above. The values obtained are shown in Table 3.

Table 3

| Catalyst sample | $\Delta$ T required for 70% EB conversion (°C) |
|---|---|
| 5 (Pt) | 0.0 |

(continued)

| Catalyst sample | Δ T required for 70% EB conversion (°C) |
|---|---|
| 6 (Pt/Cu) | 1.1 |
| 7 (Pt/Ag) | -0.8 |

The results show that there is no significant loss in activity in comparison to the platinum only catalyst, and in the silver containing catalyst there is in fact a slightly higher activity, which is a beneficial result. Thus the activity is not significantly affected and indeed for one embodiment, the activity slightly increases.

Example 12

Activity Testing of Catalysts 1, 3, 8 and 9

The catalyst samples produced in examples 1, 3, 8 and 9 were activity tested as described above. The results are shown in table 4.

Table 4

| Catalyst sample | Δ T required for 70% EB conversion (°C) |
|---|---|
| 1 (Pt) | 0.0 |
| 3(Pt/0.01%wt Cu) | 3.5 |
| 8 (Pt/0.005%wt Cu) | 1.7 |
| 9 (Pt/0.002wt% Cu) | 2.5 |

The results obtained show that all the catalysts are comparable in terms of ethylbenzene dealkylation activity. It is also clear that the ethylbenzene conversion is not a linear function of the copper loading, and cannot be interpolated from the two most extreme cases with and without the copper present in the catalyst composition.
Tables 5, 6 and 7 below compare activities of various catalyst examples. In each table, the catalysts are compared to comparative example 1 - the platinum only case. This is taken as a comparative reference and its performance is rated as zero for each parameter tested. All relative performance parameters are taken at identical ethylbenzene conversion of 60 wt% using the definitions as laid out above.

Comparison of Catalyst performance activity testing of catalyst samples 1 to 4

The test results for the activity testing conducted for catalyst samples 1, 2, 3 and 4 are summarised in table 5.
Table 5 shows that addition of nickel, copper or silver to the platinum catalyst decreased the transalkylation related catalytic activity, at identical ethylbenzene conversion. Xylene losses, toluene produced, and $C_9^+$ aromatics yields are lower than those observed in the platinum only catalyst.
In the cases of silver and copper addition the obtained benzene selectivity is comparable, the difference is not significant. However, the benzene purity is lower in all bimetallic samples. The highest purity among the bimetallic catalysts is measured in the silver containing sample.

Table 5

| Catalyst sample | Xylene Losses (%) | Toluene yield (wt%) | $C_{9+}$ yield (wt%) | Benzene selectivity (mol%) | Benzene purity (%wt) |
|---|---|---|---|---|---|
| 1 (Pt) | 0 | 0 | 0 | 0 | 0 |
| 2 (Pt/Ni) | -37 | -41 | -26 | -35 | -66 |
| 3 (Pt/Cu) | -51 | -58 | -44 | -4 | -74 |
| 4 (Pt/Ag) | -38 | -40 | -29 | -9 | -24 |

Thus all catalysts in accordance with the present invention beneficially reduce xylene losses, toluene yield and $C_{9+}$ yield compared to the Pt-only sample. Reducing such losses would normally result in a significant reduction in benzene selectivity and purity to levels of around -200%. Whilst benzene selectivity and purity for embodiments of the present invention are reduced to a certain extent, the degree of reduction is much less than would be expected.

Based on the results obtained it is shown that the addition of silver, in particular, allows the simultaneous decrease of the transalkylation activity while largely retaining the high benzene selectivity, and whilst not reducing the benzene purity considerably.

It is note worthy that a similar - beneficial - decrease in the transalkylation activity for the comparative platinum only containing catalyst of Example 1 could only be achieved at a significantly lower ethylbenzene conversion level. Conversion levels of the Pt-only sample would need to be reduced by approximately 35% to around 35% to achieve a similar reduction in transalkylation activity as the copper containing catalyst operating at 70% conversion, and reduced by 20% to around 50% to achieve a similar reduction in transalkylation activity as the silver containing catalyst operating at 70% conversion.

Specifically, for the platinum only containing catalyst of Example 1 to achieve a comparable low level of transalkylation activity to that of the silver containing bimetallic sample the ethylbenzene conversion would have to be decreased by 80%.

Comparison of Catalyst performance activity testing of catalyst samples 1, 6 and 7

The relative performance of the catalysts samples produced in Examples 1, 6 and 7 were tested in the procedure described herein. The data obtained is shown in table 6.

Table 6

| Catalyst sample | Xylene Losses (%) | Toluene yield (wt%) | $C_9^+$ yield (wt%) | Benzene selectivity (mol%) | Benzene purity (%wt) |
|---|---|---|---|---|---|
| 1 (Pt) | 0 | 0 | 0 | 0 | 0 |
| | | | | | |
| 6 (Pt/Cu) | -40 | -51 | -38 | -14 | -88 |
| 7 (Pt/Ag) | -34 | -33 | -25 | -6 | 9 |

The results in table 6 show the effects of the higher calcination temperature (600°C) employed in examples 6 and 7. The two higher calcination temperature examples beneficially show a lower transalkylation performance compared to example 1. In terms of benzene selectivity, all the examples in table 6 perform consistently, there are no significant differences, and only a minor loss in benzene selectivity is observed.

In the silver containing sample the benzene purity is improved above that of even catalyst 1. Thus in especially preferred embodiments of the present invention containing silver, not only is the transalkylation activity suppressed to a greater extent than that of the Pt-only catalyst but the activity is also improved. This is particularly surprising because normally lower benzene purity is observed when higher calcination temperatures are used, due to the sintering of the metallic phase promoting, among other reactions, the saturation of aromatic modules. As such, it would be typical to look to manipulation of the support to improve the benzene purity and not addition of a metallic component.

Based on the results obtained, Example 7 offers the advantageous lower transalkylation activity, also coupled with similar or higher benzene selectivity and purity above that of the platinum only version.

Comparison of Catalyst performance activity testing of examples 1, 3, 8 and 9

Table 7. Relative performance parameters of example 2, 3, 8, and 9 compared to Example 1

| | Xylene losses (%) | Toluene yield (wt%) | $C_9^+$ yield (wt%) | Benzene selectivity (mol%) | Benzene purity (%wt) |
|---|---|---|---|---|---|
| Example 1 (Pt) | 0 | 0 | 0 | 0 | 0 |

(continued)

|  | Xylene losses (%) | Toluene yield (wt%) | $C_9^+$ yield (wt%) | Benzene selectivity (mol%) | Benzene purity (%wt) |
|---|---|---|---|---|---|
| Example 3 (Pt/ 0.01wt%Cu) | -51 | -58 | -44 | -4 | -74 |
| Example 8 (Pt/ 0.005wt%Cu) | -29 | -38 | -19 | -54 | -58 |
| Example 9 (Pt/ 0.002wt%Cu) | -24 | -33 | -19 | -53 | -88 |

Table 7 shows a systematic increase in xylene losses with decreasing amounts of copper present in the formulation. Consistent with this, the toluene yield and heavy aromatics yield is also increasing with decreasing amount of copper present.

Benzene selectivity does not improve with lowering the copper content, though it is the best at the higher amount and decreased considerably at the lower levels of copper. The benzene purity is low of all these samples. This clearly demonstrates that with copper/platinum formulations compared to the platinum only formulation, it is not possible to get both lower transalkylation activity, high benzene selectivity, and benzene purity at the same time; all measured at identical ethyl benzene conversion levels.

However, the lowest xylene losses, toluene yield and $C_{9+}$ aromatics yield can be achieved with the copper containing samples. This is advantageous in certain situations or unit configurations/layouts, where benzene purity is of lesser importance. For such embodiments, an increased amount of copper is preferred.

Based on the examples above it can be seen that the inclusion of a additional metal in the base platinum containing catalyst can have a positive effect by reducing the transalkylation activity. One embodiment of the platinum and silver containing composition also surprisingly shows improved benzene purity.

## Claims

1. A catalyst composition which comprises:

    a) a carrier which comprises a pentasil zeolite of at least 20wt% on the basis of the total carrier and being in its $H^+$ form;
    b) platinum in an amount in the range of from 0.001 to 0.1 wt%, on the basis of the total catalyst; and,
    c) at least one additional metal selected from: nickel, copper and silver in an amount in the range from 0.0001 to 0.1 wt% on the basis of the total catalyst.

2. Catalyst composition as claimed in claim 1, wherein the additional metal selected is silver.

3. Catalyst composition as claimed in claim 1 or claim 2, wherein the silver is in an amount in the range from 0.001 to 0.05 wt%, preferably 0.001 - 0.03wt% on the basis of the total catalyst.

4. Catalyst composition as claimed in any proceeding, wherein the pentasil zeolite is of one of the MFI, MTW or FER configurations.

5. Catalyst composition as claimed in claim 4, wherein the zeolite has an MFI configuration and is ZSM-5.

6. Catalyst composition as claimed in claim 5, wherein the ZSM-5 has a SAR in the range of from 20 to 50, preferably 20 to 40, and is in the carrier in an amount of from 20 to 50 wt%, preferably from 25 to 45 wt%.

7. Catalyst composition as claimed in any preceding claim, comprising alumina and silica and having a bulk alumina to silica ratio in the range of from 20 to 150; at least 30wt% of a binder selected from silica, zirconia and titania.

8. Catalyst composition as claimed in claim 7, wherein the silica mean particle size is in the range of from 2 to 60 $\mu$m.

9. A process for the preparation of a catalyst composition as claimed in claim 1, which comprises combining at least 20 wt% of pentasil zeolite, shaping the resulting mixture, if desired, and compositing within the range of from 0.001 to 0.1 wt% platinum, and in the range of from 0.0001 to 0.1 wt% of at least one additional metal selected from: nickel, copper, or silver.

10. A process as claimed in claim 9, wherein following addition of the metals, the catalyst composition is calcined at a temperature in the range of from 550 °C to 650 °C preferably where the second metal is silver.

11. An ethylbenzene dealkylation process which comprises contacting in the presence of hydrogen a feedstock which comprises ethylbenzene, with a catalyst composition as claimed in any one of claims 1 to 8 or as prepared by the process as claimed in claim 9 or 10.

12. Catalyst composition as claimed in claim 1, preparation process as claimed in claim 9 or 10, and an ethylbenzene dealkylation process as claimed in claim 11, which are substantially as described in any one of the Examples herein.

# EP 2 022 564 A1

European Patent Office

**EUROPEAN SEARCH REPORT**

Application Number

EP 07 11 3573

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 4 899 011 A (CHU YUNG F [US] ET AL) 6 February 1990 (1990-02-06) <br> * column 2, lines 54-62 * <br> * column 3, lines 11-24 * <br> * column 5, lines 12-16 * <br> * column 6, lines 9-31,42-49 * <br> * column 7, lines 16-22,28-33,46-65 * <br> * column 8, lines 16-22,26-68 * <br> ----- | 1,4,5, 9-12 | INV. <br> B01J29/44 <br> B01J29/46 <br> C07C4/18 |
| X | US 6 051 744 A (NACAMULI GERALD J [US] ET AL) 18 April 2000 (2000-04-18) <br> * abstract * <br> * column 1, lines 6-9 * <br> * column 5, lines 2-13,49-52 * <br> * column 7, lines 6-21,37-43 * <br> * column 8, lines 48-62 * <br> * column 9, lines 1-13 * <br> * column 12, lines 12-21,35-44 * <br> * claims 1,4,6-9,13,15 * <br> ----- | 1,4-6, 9-12 | |
| D,X | EP 1 495 805 A (TORAY INDUSTRIES [JP]) 12 January 2005 (2005-01-12) <br> * page 5, line 16 * <br> * page 7, paragraphs 33,35,36 * <br> * page 8, paragraph 38 * <br> * claims 1-5,7,11 * <br> ----- | 1-12 | TECHNICAL FIELDS SEARCHED (IPC) <br><br> B01J <br> C07C |
| X | WO 2006/103754 A (SUED CHEMIE CATALYSTS JAPAN IN [JP]; SHIOYA YASUSHI [JP]; MIYAKI YOSHI) 5 October 2006 (2006-10-05) <br> * claims 1,2 * <br> * examples 1,7 * <br> * page 3, paragraphs 20,21 * <br> * page 4, paragraphs 24,26,31,32 * <br> & EP 1 872 852 A (SUED CHEMIE CATALYSTS JAPAN IN [JP]) 2 January 2008 (2008-01-02) <br> ----- <br><br> -/-- | 1,4,5,9 | |

The present search report has been drawn up for all claims

| Place of search | Date of completion of the search | Examiner |
|---|---|---|
| Munich | 25 March 2008 | Jourdan, Angelika |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**European Patent Office**

## EUROPEAN SEARCH REPORT

Application Number

EP 07 11 3573

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 4 946 813 A (SHUM VICTOR K [US]) 7 August 1990 (1990-08-07) * claims 1-3,5,8 * * column 4, lines 59-62 * * column 6, line 51 - column 7, line 6 * * column 7, lines 42-53 * * example 1 * | 1,4,9 | |
| A | US 3 992 468 A (COSYNS JEAN ET AL) 16 November 1976 (1976-11-16) * claims 1,6 * * example 6 * | 1-12 | |
| A | US 5 456 822 A (MARCILLY CHRISTIAN [FR] ET AL) 10 October 1995 (1995-10-10) * claims 1,2,7 * * column 3, lines 6-23,42-48,53-62 * * column 4, lines 5-12 * | 1-10 | |
| A | L.MELO ET AL.: "Acetone transformation over Pt Cu/H[Al]ZSM5 catalysts. Effect of copper content" CATALYSIS LETTERS, vol. 78, no. 1-4, March 2002 (2002-03), pages 57-63, XP002473713 * page 58, left-hand column, paragraph 3-6 * * page 59; table 1 * | 1-10 | TECHNICAL FIELDS SEARCHED (IPC) |

The present search report has been drawn up for all claims

| Place of search | Date of completion of the search | Examiner |
|---|---|---|
| Munich | 25 March 2008 | Jourdan, Angelika |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 07 11 3573

This annex lists the patent family members relating to the patent documents cited in the above-mentioned European search report.
The members are as contained in the European Patent Office EDP file on
The European Patent Office is in no way liable for these particulars which are merely given for the purpose of information.

25-03-2008

| Patent document cited in search report | | Publication date | Patent family member(s) | | Publication date |
|---|---|---|---|---|---|
| US 4899011 | A | 06-02-1990 | CA | 1271782 A1 | 17-07-1990 |
| | | | DE | 3771232 D1 | 14-08-1991 |
| | | | EP | 0234684 A1 | 02-09-1987 |
| | | | IN | 169198 A1 | 14-09-1991 |
| | | | JP | 8016074 B | 21-02-1996 |
| | | | JP | 62169736 A | 25-07-1987 |
| | | | ZA | 8700290 A | 31-08-1988 |
| US 6051744 | A | 18-04-2000 | AT | 260227 T | 15-03-2004 |
| | | | AU | 768545 B2 | 18-12-2003 |
| | | | AU | 1736100 A | 03-07-2000 |
| | | | CA | 2320698 A1 | 22-06-2000 |
| | | | DE | 69915036 D1 | 01-04-2004 |
| | | | DE | 69915036 T2 | 30-09-2004 |
| | | | EP | 1056695 A1 | 06-12-2000 |
| | | | ES | 2214904 T3 | 16-09-2004 |
| | | | GC | 0000116 A | 29-06-2004 |
| | | | TW | 523501 B | 11-03-2003 |
| | | | WO | 0035838 A1 | 22-06-2000 |
| EP 1495805 | A | 12-01-2005 | AT | 365072 T | 15-07-2007 |
| | | | CA | 2473066 A1 | 08-01-2005 |
| | | | DE | 602004007066 T2 | 27-09-2007 |
| | | | ES | 2287654 T3 | 16-12-2007 |
| | | | KR | 20050006043 A | 15-01-2005 |
| | | | SG | 108993 A1 | 28-02-2005 |
| | | | TW | 263630 B | 11-10-2006 |
| | | | US | 2005010074 A1 | 13-01-2005 |
| WO 2006103754 | A | 05-10-2006 | EP | 1872852 A1 | 02-01-2008 |
| EP 1872852 | A | 02-01-2008 | WO | 2006103754 A1 | 05-10-2006 |
| US 4946813 | A | 07-08-1990 | NONE | | |
| US 3992468 | A | 16-11-1976 | CA | 1067104 A1 | 27-11-1979 |
| | | | DE | 2508291 A1 | 04-09-1975 |
| | | | IT | 1033322 B | 10-07-1979 |
| | | | JP | 50121233 A | 23-09-1975 |
| | | | NL | 7502435 A | 03-09-1975 |
| US 5456822 | A | 10-10-1995 | NONE | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 0018498 A
- EP 0425712 A
- WO 0038834 A
- EP 018498 A1
- US 3992468 A
- EP 1495805 A

- US 3702886 A
- US 4511547 A, Iwayama
- US 6949181 B2
- US 5242676 A
- US 4088605 A

**Non-patent literature cited in the description**

- **TOPPI.** *Journal of Catalysis,* 2002, vol. 210, 431-444
- Atlas of Zeolite framework types. **BAERLOCHER et al.** Structure Commission of the International Zeolite Association. Elsevier, 2001

- **YU et al.** *Microporous and Mesoporous Materials,* 2006, vol. 95, 234-240